(19) Europäisches Patentamt
European Patent Office
Office européen des brevets

(11) Numéro de publication: **0 133 127**
**B1**

# (12) FASCICULE DE BREVET EUROPÉEN

(45) Date de publication du fascicule du brevet: **18.03.87**

(21) Numéro de dépôt: **84401560.2**

(22) Date de dépôt: **25.07.84**

(51) Int. Cl.⁴: **C 07 F 9/50**, B 01 J 31/24, C 07 C 103/46

(54) **Nouvelles phosphines chirales sulfonées, leur préparation et leur emploi en catalyse asymétrique.**

(30) Priorité: **28.07.83 FR 8312468**

(43) Date de publication de la demande: **13.02.85 Bulletin 85/7**

(45) Mention de la délivrance du brevet: **18.03.87 Bulletin 87/12**

(84) Etats contractants désignés: **AT BE CH DE FR GB IT LI LU NL SE**

(56) Documents cité:
**FR-A-2 230 654**
**FR-A-2 349 562**
**FR-A-2 499 978**

(73) Titulaire: **RHONE- POULENC SANTE, Les Miroirs 18 Avenue d'Alsace, F-92400 Courbevoie Cedex (FR)**

(72) Inventeur: **Dang, Tuan- Phat, 29, rue du Commandant Faurax, F-69006 Lyon (FR)**
Inventeur: **Jenck, Jean, 4, avenue de la Paix Chalampé, F-68490 Ottmarsheim (FR)**
Inventeur: **Morel, Didier, 3, rue Jean- Jaurès, F-91700 Villiers sur Orge (FR)**

(74) Mandataire: **Pilard, Jacques, RHONE- POULENC RECHERCHES Service Brevets Pharma 25, Quai Paul Doumer, F-92408 Courbevoie Cedex (FR)**

**EP 0 133 127 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

La présente invention concerne de nouvelles phosphines chirales sulfonées optiquement actives de formule générale:

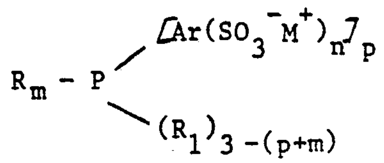

(I)

leur préparation et leur utilisation en synthèse asymétrique en chimie organique.

Dans le brevet français 2 116 905 sont décrites des phosphines asymétriques qui permettent d'obtenir des complexes de métaux de transition à coordinats optiquement actifs particulièrement utiles pour hydrogéner les composés insaturés en composés saturés doués d'activité optique. Cependant de telles phosphines ne permettent pas d'obtenir des rendements quantitatifs en un seul des isomères.

Afin d'accroître la sélectivité d'hydrogénation asymétrique des acides et esters acryliques substitués précurseurs d'aminoacides, il a été proposé, dans le brevet français 2 230 654, d'utiliser des stéréoisomères optiquement actifs de trans-bis(phosphinométhyl)-1,2 cyclobutanes pour la préparation de complexes du rhodium. Cependant, l'utilisation de ces phosphines ne permet pas un recyclage du catalyseur.

Il a maintenant été trouvé et c'est ce qui fait l'objet de la présente invention que les phosphines chirales sulfonées de formule générale (I) permettent d'obtenir des complexes de métaux de transition solubles dans l'eau utilisables pour la mise en oeuvre de synthèses asymétriques et qui permettent un recyclage du catalyseur.

Les phosphines chirales sulfonées, qui présentent l'avantage d'être solubles dans l'eau, permettent de rendre solubles dans l'eau les coordinnats de complexes métalliques catalytiques. Il est ainsi possible d'effectuer la réaction envisagée dans un milieu comprenant de l'eau et un solvant organique approprié. Dans ces conditions, en fin de réaction, le produit de réaction se retrouvera dans la phase organique et le système catalytique dans la phase aqueuse qui pourra être recyclée.

Dans la formule générale (I),

- R représente un radical alcoyle contenant 1 à 12 atomes de carbone en chaîne droite ou ramifiée, cycloalcoyle contenant 3 à 6 atomes de carbone et éventuellement substitué par 1 ou 2 radicaux alcoyles contenant 1 à 4 atomes de carbone, cycloalcoylalcoyle contenant 4 à 10 atomes de carbone et éventuellement substitué par un radical alcoyle contenant 1 à 4 atomes de carbone, phényle ou phénylalcoyle dont la partie alcoyle contient 1 à 4 atomes de carbone, binaphtyle ou binaphtylalcoyle dont la partie alcoyle contient 1 à 4 atomes de carbone, les radicaux alcoyles, cycloalcoyles, phényles, binaphtyles et les portions alcoyles des autres radicaux étant éventuellement substitués par un radical de formule générale:

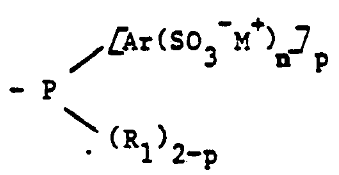

(II)

- $R_1$ représente un radical alcoyle contenant 1 à 12 atomes de carbone en chaîne droite ou ramifiée, cycloalcoyle contenant 3 à 6 atomes de carbone, phényle ou phénylalcoyle dont la partie alcoyle contient 1 à 4 atomes de carbone,
- Ar représente un radical phényle ou naphtyle éventuellement substitué par un ou plusieurs radicaux alcoyles ou alcoyloxyles contenant 1 à 4 atomes de carbone
- $M^+$ représente un proton ou un ion dérivé d'un métal alcalin ou alcalino-terreux,
- n représente un nombre entier égal à 1 ou 2

- m représente un nombre entier égal à 0, 1 ou 2, et
- p représente un nombre entier égal à 1 ou 2, étant entendu que p + m est inférieur ou égal à 3,

étant entendu que les radicaux R et $R_1$ peuvent être chiraux ou achiraux, et que

- lorsque l'un des radicaux R et $R_1$ est chiral, p et m peuvent être égaux à 1 ou 2
- lorsque les radicaux R et $R_1$ sont achiraux, p et m sont nécessairement égaux à 1, les radicaux R et $R_1$ étant alors différents de façon que la substitution du ou des atome(s) de phosphore du produit de formule générale (I) soit dissymétrique
- lorsque m est égal à 0, les radicaux $R_1$ sont différents de façon que la substitution de l'atome de phosphore du produit de formule générale (I) soit dissymétrique.

D'un intérêt tout particulier sont les produits de formule générale (I) dans laquelle:

R représente un radical alcoyle contenant 2 à 6 atomes de carbone en chaîne droite ou ramifiée éventuellement substitué par un radical de formule générale (II), un radical cycloalcoyle substitué par 1 ou 2 radicaux alcoyles contenant 1 à 4 atomes de carbone, un radical cycloalcoylalcoyle contenant 6 à 10 atomes de carbone, la partie cycloalcoyle contenant 4 à 6 atomes de carbone et étant substituée par un radical alcoyle contenant 1 à 4 atomes de carbone lui-même substitué par un radical de formule générale (II),

$R_1$ représente un radical phényle,

Ar représente un radical phényle,

M+ représente un ion de métal alcalin

n est égal à 1 ou 2

m est égal à 1, et

p est égal à 1 ou 2.

Selon la présente invention, les nouvelles phosphines de formule générale (I) peuvent être obtenues par sulfonation d'une phosphine de formule générale:

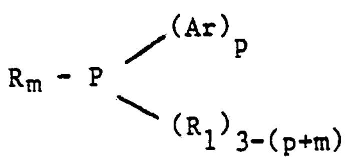

(III)

dans laquelle R, $R_1$, Ar, sont définis comme précédemment, m et p étant éqaux à 1 ou 2 et p + m étant $\leqslant 3$ l'un des symboles R et $R_1$ étant chiral.

Généralement, la sulfonation est effectuée au moyen d'un oléum à une température voisine de 20°C. Il est particulièrement avantageux d'utiliser un oléum contenant 20 à 70 % d'anhydride sulfurique.

Parmi les agents de sulfonation qui peuvent aussi être utilisés pour la mise en oeuvre du procédé selon l'invention peuvent être cités l'acide chlorosulfonique, les dérivés de l'anhydride sulfurique, tel que les mélanges anhydride sulfurique-dioxanne ou le sulfate de bis-triméthylsilyle.

Généralement, la sulfonation du radical Ar de la phosphine de formule générale (III) s'effectue en position méta.

Lorsque dans la phosphine de formule générale (III) Ar et $R_1$ représentent chacun un radical phényle, il peut se produire une monosulfonation du radical Ar ou $R_1$ ce qui conduit à un produit de formule générale (I) dans laquelle m = 1, n = 1 et p = 1 ou une monosulfonation de chacun des radicaux Ar et $R_1$ ce qui conduit à un produit de formule générale (I) dans laquelle m = 1, n = 1 et p = 2. Généralement, la sulfonation conduit à un mélange de phosphines sulfonées qui sont dénommées phosphine monosulfonée, phosphine disulfonée, diphosphine trisulfonée et diphosphine tétrasulfonée.

Lorsque la phosphine de formule générale (III) contient 2 atomes de phosphore, il se forme en général un mélange de diphosphine trisulfonée et de diphosphine tétrasulfonée, la diphosphine tétrasulfonée se formant majoritairement.

Lorsque la phosphine de formule générale (III) est chirale, le procédé de sulfonation conduit à une phosphine sulfonée chirale.

Lorsque dans la phosphine de formule générale (III) le radical R est racémique, la sulfonation conduit à une phosphine sulfonée de formule générale (I) racémique qui peut être dédoublée, par exemple, au moyen d'une base optiquement active selon les techniques habituelles de dédoublement.

Lorsque dans la phosphine de formule générale (III), les radicaux R et $R_1$ étant achiraux et le ou les atome(s) de phosohore sont dissymétriquement substitués, c'est-à-dire lorsque la phosphine de formule générale (III) est prochirale ou racémique, la sulfonation conduit à une phosphine sulfonée racémique qui peut être dédoublée, par exemple, au moyen d'une base optiquement active selon les techniques habituellee de dédoublement.

Quel que soit le procédé utilisé, la phosphine sulfonée obtenue sous forme acide peut être transformée en sel de métal alcalin par action d'une base telle qu'un hydroxyde de métal alcalin.

Selon le procédé de préparation des phosphines sulfonées de formule générale (I), il est possible d'obtenir chacune des formes R et S. Chacune de ces formes fait partie de l'invention. Cependant doit être aussi considéré comme faisant partie de l'invention les mélanges des formes chirales renfermant une proportion majoritaire d'une des formes.

Les phosphines chirales sulfonées selon la présente invention présentent un intérêt tout particulier en chimie organique dans des procédés de synthèse asymétrique.

Plus particulièrement, les phosphines chirales sulfonées peuvent être utilisées pour réaliser des réactions asymétriques d'hydrogénation, d'hydroformylation, d'hydrocarboxylation, d'isomérisation, d'oligomérisation, de polymérisation, d'oxydation, de télomérisation ou d'hydrocyanation.

Par exemple, les phosphines chirales sulfonées selon l'invention, utilisées pour la préparation de complexes du rhodium, permettent l'hydrogénation asymétrique d'acides acryliques substitués précurseurs d'aminoacides ou de leurs esters. Sous le terme acides et esters acryliques substitués on entend l'ensemble des composés dont la formule dérive de celle de l'acide acrylique et de ses esters en substituant au plus deux des atomes d'hydrogène portés par les atomes de carbone éthylénique de la manière suivante:

- un des atomes d'hydrogène est substitué par un groupement amine, celui-ci pouvant être primaire ou secondaire. Le groupement aminé peut être substitué par un groupement acyle tel qu'acétyle ou benzoyle
- un autre des atomes d'hydrogène des carbones éthyléniques peut être remplacé par un groupement alcoyle (méthyle, éthyle, isopropyle, isobutyle), cycloalcoyle (cyclopentyle, cyclohexyle), aromatique (phényle, naphtyle) ou hétérocyclique (furyle, pyrannyle, benzopyrannyle, pyrrolyle, pyridyle, indolyle).

Parmi les acides et esters acryliques substitués précurseurs d'acide aminés, on peut citer l'acide N-acétyl α-amino β-phénylacrylique, l'acide N-benzoyl α-amino β-phénylacrylique, dans lesquels le noyau phényle est éventuellement substitué par un ou plusieurs radicaux alcoyles, alcoyloxy ou hydroxy, l'acide N-acétyl α-amino β-indolylacrylique, l'acide N-benzoyl α-amino β-indolylacrylique, l'acide N-acétyl α-amino β-isobutyl acrylique.

L'hydrogénation asymétrique sélective est effectuée en utilisant comme catalyseurs les complexes du rhodium tel que $[Rh(cyclooctadiène\text{-}1,5)Cl]_2$ ligandés par les phosphines chirales sulfonées de formule générale (I).

L'hydrogénation s'effectue généralement à une température comprise entre 20 et 100° C et sous une pression d'hydrogène comprise entre 0,5 et 50 bars.

Le complexe de rhodium est utilisé de telle manière que le rapport entre le nombre d'atomes de rhodium présent dans le complexe et le nombre de moles du composé à hydrogéner soit compris entre 0,1 et 0,0001.

Le procédé d'hydrogénation est mis en oeuvre, de préférence, dans un mélange d'eau et d'un solvant organique non miscible tel que l'acétate d'éthyle.

Par hydrogénation, il est donc possible d'obtenir avec de bons rendements les différents stéréoisomères d'aminoacides.

L'hydroformylation asymétrique sélective peut être réalisée en utilisant comme catalyseurs des complexes du rhodium ligandés par des phosphines chirales sulfonées de formule générale (I).

L'hydroformylation s'effectue généralement à une température compriee entre 20 et 150° C et soue une pression d'hydrogène et d'oxyde de carbone comprise entre 1 et 200 bars.

Le complexe du rhodium est utilisé de telle manière que le rapport entre le nombre d'atomes de phosphore de la phosphine et le nombre d'atomes de rhodium présent dans le complexe soit compris entre 2 et 100 lorsque l'on utilise une monophosphine et entre 2 et 10 lorsque l'on utilise une diphospine.

Les exemples suivants, donnés à titre non limitatif, illustrent la présente invention.

**Exemple 1**

Une solution de 1 g de (±) trans bis(diphénylphosphinométhylène)-1,2 cyclobutane dans 2 $cm^3$ d'acide sulfurique à 66° Baumé est versée dans 19 g d'oléum contenant 20 % d'anhydride sulfurique préalablement refroidi à 0° C. La température étant maintenue à 20° C au moyen d'un bain thermostaté, la réaction se poursuit pendant 27 heures, le déroulement de la réaction étant contrôlée par examen en RMN du $P^{31}$ de fractions aliquotes.

Le mélange réactionnel est ensuite versé avec précaution dans 30 g de glace. Après neutralisation par addition de soude aqueuse à 50%, le précipité de sulfate de sodium (59,7 g) est séparé par filtration. Après refroidissement à 0° C, le mélange réactionnel est à nouveau filtré puis additionné de méthanol. Après filtration pour éliminer la totalité du sulfate de sodium, le filtrat est concentré à sec. On obtient ainsi 0,58 g de (±) trans bis(diphénylphosphinométhylène)-1,2 cyclobutane sulfoné.

L'analyse par RMN du $P^{31}$ sur le produit tel quel puis oxydé par l'eau oxygénée montre que le produit obtenu contient:

62 % de diphosphine tétrasulfonée
34% de diphosphine trisulfonée
4% d'oxydes de phosphines

Les caractéristiques du spectre RMN du $P^{31}$ sont les suivantes:

a) solvant: acide sulfurique; référence: acide phosphorique:

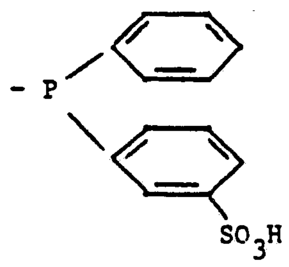

δ = 5,5 ppm

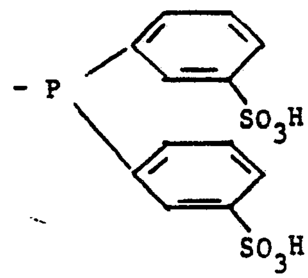

δ = 6,3 ppm

b) solvant: eau; référence acide phosphorique, après oxydation par l'eau oxygénée:

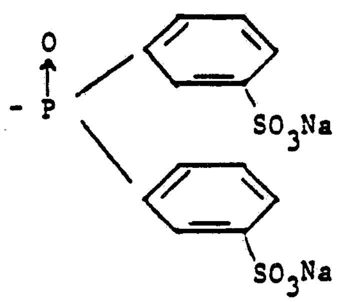

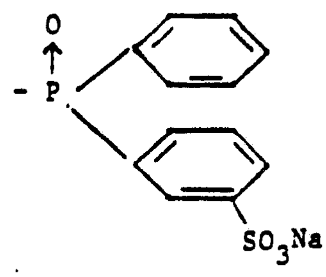

δ = 37,9 ppm: diphosphine tétrasulfonée
δ = 38,7 ppm: diphosphine trisulfonée.
Analyse élémentaire:
Trouvé : C % 39,1 x H % 3,9 x P = 6,74 x S = 11,94
S/P = 1,71 (théorie: 2 pour la phosphine tétrasulfonée: 1,5 pour la diphosphine trisulfonée).

**Exemple 2**

Une solution de 1 g de (-) trans bis(diphénylphosphino-méthylène)-1,2 cyclobutane dans 2 $cm^3$ d'acide sulfurique à 66° Baumé est versée dans 19 g d'oléum contenant 20 % d'anhydride sulfurique préalablement refroidi à 0°C. Le mélange réactionnel, placé sous un courant d'azote, est maintenu à 22°C pendant 45 heures. Le mélange réactionnel est alore versé avec précaution dans 30 g de glace puis il est neutralisé par addition de soude à 50 % tout en maintenant la température au-dessous de 25°C.

Le précipité de sulfate de sodium est séparé par filtration. Après refroidiseement du filtrat à 0°C, le sulfate de sodium est séparé par filtration puis on ajoute au filtrat 200 $cm^3$ de méthanol. Le sulfate de sodium est séparé par filtration. Le filtrat est concentré à sec. On obtient ainsi 2,32 g de (-) trans bis(diphénylphosphinométhylène)-1,2 cyclobutane sulfoné.

L'analyse par RMN du $P^{31}$ montre que le produit obtenu contient 7% d'oxydes de phosphine et 93% de phosphine sulfonée constituée de 67% de diphosphine tétra(métasulfonée) et de 33% de diphosphine tri(métasulfonée).

Le pouvoir rotatoire est: $[\alpha]_D = 11{,}6°$ (C = 0,52, eau).

**Exemple 3**

Une solution de 0,8 g de (-) (2S,3S) bis(diphénylphosphino) butane dans 1,6 $cm^3$ d'acide sulfurique à 66° Baumé est versée dans 16 g d'oléum à 20 % d'anhydride sulfurique préalablement refroidi à 0°C. Le mélange réactionnel est agité pendant 28 heures à 22°C. Après refroidissement à 0°C on ajoute, avec précaution, 25 g de glace tout en maintenant la température au-dessous de 15°C. Le mélange réactionnel est alors neutralisé (pH = 7) par addition de soude à 50 %. Le sulfate de sodium est séparé par filtration. Après refroidissement du filtrat à 0°C, le sulfate de sodium est séparé par filtration puis on ajoute au filtrat 60 $cm^3$ de méthanol. Le sulfate de sodium précipité est séparé par filtration. Le filtrat est concentré à sec. On obtient ainsi 1,6 g de (-) (2S,3S) bis (diphénylphosphino) butane sulfoné (fraction A).

Les deux derniers précipités de sulfate de sodium sont réunis et lavés par 200 $cm^3$ de méthanol. Après filtration, le filtrat méthanolique est évaporé à sec. On obtient ainsi une seconde fraction de 0,4 g de (-) (2S,3S) bis(diphénylphosphino)butane sulfoné (fraction B).

Le dosage par iodométrie montre que la fraction A contient 18% de phosphine sulfonée et que la fraction B contient 75 % de phosphine sulfonée (comptée en diphosphine tétrasulfonée).

L'analyse par RMN du $P^{31}$ montre que, pour la fraction B, le rapport phosphines/oxydes de phosphines = 76/24.

L'analyse élémentaire de la fraction B montre que le rapport S/P = 1,55.

**Exemple 4**

A une solution de 0,7 g de (-) (2S,3S) bis(diphénylphosphino) butane dans 4 $cm^3$ d'acide sulfurique à 98,5% on ajoute 3,5 $cm^3$ d'oléum à 65 % d'anhydride sulfurique. Le mélange réactionnel est agité pendant 22 heures à 22°C. Après refroidissement à -5°C on ajoute, par petites portions, 20 g de glace. Tout en maintenant la température inférieure à 10°C, le mélange réactionnel est neutralisé par addition de soude à 50%. Le précipité de sulfate de sodium est séparé par filtration. Après avoir ajouté du méthanol au filtrat, on sépare à nouveau du sulfate de sodium par filtration, puis le filtrat est concentré à sec. On obtient ainsi 0,985 g de (-) (2S,3S) bis(diphénylphosphino) butane sulfoné.

L'analyse par RMN du $P^{31}$ (solvant: eau; référence acide phosphorique) montre qu'il y a 10 % d'oxydes de phosphines (6 % $\delta$ = 43,5 ppm; 4 % $\delta$ = 44,3 ppm) et 90 % de phosphines sulfonées (72 % $\delta$ = -9,7 ppm; 15% $\delta$ = -8,9 ppm et 3% $\delta$ = -7,3 ppm).

L'analyse élémentaire indique un rapport S/P = 1,45.

Pouvoir rotatoire: $[\alpha]_D$ = -98,5° (C = 0,965 eau).

**Exemple 5**

Une solution de 0,5 g de néomenthyldiphénylphosphine dans 2 $cm^3$ d'acide sulfurique à 98% est ajoutée à 10 $cm^3$ d'oléum contenant 20 % d'anhydride sulfurique. On agite pendant 2 heures à 22°C puis on ajoute, par petites portions, avec précaution, 30 g de glace puis neutralise (pH = 7) par addition de soude à 50 % tout en maintenant la température inférieure à 15°C. Le sulfate de sodium est séparé par filtration. Au filtrat, on ajoute du méthanol puis sépare à nouveau le sulfate de sodium par filtration. Après concentration à sec du filtrat, on obtient 1,08 g de néomenthyldiphénylphosphine sulfonée.

L'analyse par RMN du $P^{31}$ montre que le produit contient 43 % d'oxydes de phosphines [$\delta$ = +20, 8 ppm (4,5 %); $\delta$ = +33,6 ppm (7,5 %); $\delta$ = 34,4 ppm (31%)] et 57 % de phosphine sulfonée [$\delta$ = -15,4 ppm (31 %); $\delta$ = -14,7 ppm (26 %)] correspondant aux 2 diastéréoisomères.

Pouvoir rotatoire: $[\alpha]_D$ = +53° (C = 0,47; méthanol).

**Exemple 6**

On dissout, sous atmosphère d'azote, 25 mg de [Rh (cyclooctadiène-1,5) Cl]$_2$ [0,1 milliatome-gramme de rhodium] et 112 mg (0,11 m.mole) de diphosphine sulfonée préparée à l'exemple 1 dans 10 $cm^3$ d'eau. Cette solution est injectée dans un réacteur d'hydrogénation contenant une suspension de 4,1 g (20 m.moles) d'acide $\alpha$-acétamidocinnamique dans 20 $cm^3$ d'acétate d'éthyle distillé.

L'hydrogénation est effectuée sous une pression de 1 bar d'hydrogène à une température voisine de 20°C. L'hydrogénation dure 1 heure 40. Le substrat de départ se solubilise au fur et à mesure de l'hydrogénation. La phase organique qui contient la N-acétylphénylalanine est séparé par décantation. Après concentration à sec, on obtient 3,9 g de N-acétylphénylalanine. On recharge 20 m.moles d'acide $\alpha$-acétamidocinnamique dans le réacteur où se trouve la solution aqueuse de catalyseur puis on recommence l'hydrogénation. L'opération est répétée 4 fois en obtenant des résultats analogues.

**Exemple 7**

On dissout, sous atmosphère d'azote, 26,2 mg de [Rh (cyclooctadiène-1,5) Cl]$_2$ (0,106 milli-atome-gramme de rhodium) et 175 mg (0,13 m.mole) de phosphine sulfonée préparée à l'exemple 2 dans 10 cm$^3$ d'eau. Cette solution est injectée dans un réacteur d'hydrogénation contenant une suspension de 4,1 g d'acide (Z) α-acétamidocinnamique dans 20 cm$^3$ d'acétate d' éthyle.

L'hydrogénation est effectuée sous une pression de 1 bar d'hydrogène à une température voisine de 20°C. Après 50 minutes de réaction, la quantité théorique d'hydrogène est absorbée. La phase organique est séparée par décantation puis elle est concentrée à sec. On obtient ainsi 3,9 g de N-acétylphénylalanine dont le pouvoir rotatoire est:

$[\alpha]_D = +23,3°$ (C = 1, éthanol).

Le produit obtenu a une pureté optique de 51%.

**Exemple 8**

On dissout, sous atmosphère d'azote, 9,8 mg de [Rh (cyclooctadiène-1,5) Cl]$_2$ (0,04 milli-atome-gramme de rhodium) et 71 mg (1,04 m.mole) de diphosphine sulfonée préparée à l'exemple 2 dans 10 cm$^3$ d'eau. Cette solution est injectée dans un réacteur d'hydrogénation contenant 4,38 g (20 m.moles) de (Z) α-acétamidocinnamate de méthyle dans 20 cm$^3$ d'acétate d'éthyle.

L'hydrogénation est effectuée sous une pression de 1 bar d'hydrogène à une température voisine de 20°C. L'hydrogénation dure 70 minutes.

La phase organique est séparée par décantation puis est concentrée à sec. On obtient ainsi 3,8 g d'ester méthylique de la N-acétylphénylalanine dont le pouvoir rotatoire est:

$[\alpha]_D = -20,2°$ (C = 1, chloroforme).

Le produit obtenu a une pureté optique de 20 %, le pouvoir rotatoire de l'ester méthylique de la N-acétylphénylalanine de référence étant $[\alpha]_D = 101,3°$ (chloroforme) d'après J. Organomet. Chem., 121. 249 (1976).

**Exemple 9**

Dans une ampoule de verre, on charge 788 mg d'α-acétamidocinnamate de méthyle (3,6 m.moles), 8,8 mg de [Rh(cyclooctadiène-1,5) Cl]$_2$ (0,036 milli-atome-gramme de rhodium) et 53 mg de diphosphine sulfonée préparée à l'exemple 3 (fraction B). Après avoir purgé à l'azote, on injecte 10 cm$^3$ d'acétate d'éthyle et 5 cm$^3$ d'eau. On introduit l'ampoule de verre non fermée dans un autoclave puis on purge avec un courant d'hydrogène. L'autoclave est agité pendant 17 heures sous une pression de 10 bars d'hydrogène. Après avoir purgé par un courant d'azote, la phase organique supérieure est soutirée puis concentrée à sec. On obtient ainsi 0,58 g d'ester méthylique de N-acétylphénylalanine pur (d'après l'analyse par chromatographie en phase vapeur) dont le pouvoir rotatoire est:

$[\alpha] = -80,8°$ (C = 1, chloroforme).

Le produit obtenu a une pureté optique de 79,8%.

L'ampoule de verre est rechargée avec 788 mg d'α-acétamidocinnamate de méthyle et 10 cm$^3$ d'acétate d'éthyle. Après avoir purgé l'autoclave par de l'hydrogène, celui-ci est agité pendant 17 heures sous une pression d'hydrogène de 10 bars. Après avoir purgé par un courant d'azote, la phase organique supérieure est soutirée puis concentrée à sec. On obtient ainsi 0,55 g dester méthylique de la N-acétylphénylalanine pur (d'après l'analyse par chromatographie en phase vapeur) dont le pouvoir rotatoire est:

$[\alpha]_D = -86,3°$ (C = 1, chloroforme).

Le produit obtenu a une pureté optique de 85%.

On effectue un second recyclage du catalyseur dans les conditions décrites ci-dessus mais en chargeant 1,6 g d'α-acétamidocinnamate de méathyle et 10 cm$^3$ d'acétate d'éthyle. On obtient ainsi, avec un taux de transformation de 100 %, 1,33 g d'ester méthylique de la N-acétylphénylalanine pur dont le pouvoir rotatoire est:

$[\alpha]_D = -87,3°$ (C = 1, chloroforme).

Le produit obtenu a une pureté optique de 86%.

**Exemple 10**

Dans une ampoule en verre, on charge 740 mg (3,6 m.moles) d'acide α-acétamidocinnamique, 8 mg de [Rh (cyclooctadiène-1,5) Cl]$_2$ (0,036 milli-atome-gramme de rhodium), 52 mg de diphosphine sulfonée préparée à l'exemple 4 et 7 mg de borofluorate d'argent. Après avoir purgé par un courant d'azote, on injecte 10 cm$^3$

d'acétate d'éthyle et 5 $cm^3$ d'eau.

On introduit l'ampoule de verre non fermée dans un autoclave, puis on purge par un courant d'hydrogène. L'autoclave est agité pendant 17 heures sous une pression d'hydrogène de 10 bars. Après avoir purgé par un courant d'azote, la phase organique supérieure est soutirée puis concentrée à sec. On obtient ainsi 0,71 g de N-acétylphénylalanine dont le pouvoir rotatoire est:

$[\alpha] = -38,2°$ (C = 1, éthanol).

Le produit obtenu a une pureté optique de 83%.

**Revendiations** pour les Etats contractans BE, CH, DE, FR, GB, IT, LI, LU,

NL, SE

1 - Une nouvelle phosphine chirale sulfonée optiquement active caractérisée en ce qu'elle répond à la formule qénérale:

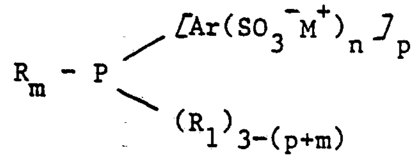

(I)

dans laquelle:

- R représente un radical alcoyle contenant 1 à 12 atomes de carbone en chaîne droite ou ramifiée, cycloalcoyle contenant 3 à 6 atomes de carbone et éventuellement substitué par 1 ou 2 radicaux alcoyles contenant 1 à 4 atomes de carbone, cycloalcoylalcoyle contenant 4 à 10 atomes de carbone et éventuellement substitué par un radical alcoyle contenant 1 à 4 atomes de carbone, phényle ou phénylalcoyle dont la partie alcoyle contient 1 à 4 atomes de carbone, binaphtyle ou binaphtylalcoyle dont la partie alcoyle contient 1 à 4 atomes de carbone, les radicaux alcoyles, cycloalcoyles, phényles, binaphtyles et les portions alcoyles des autres radicaux étant éventuellement substitués par un radical de formule générale:

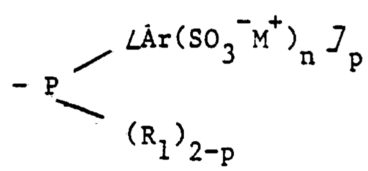

- $R_1$ représente un radical alcoyle contenant 1 à 12 atomes de carbone en chaîne droite ou ramifiée, cycloalcoyle contenant 3 à 6 atomes de carbone, phényle ou phénylalcoyle dont la partie alcoyle contient 1 à 4 atomes de carbone,

- Ar représente un radical phényle ou naphtyle éventuellement substitué par un ou plusieurs radicaux alcoyles ou alcoyloxyles contenant 1 à 4 atomes de carbone.

- $M^+$ représente un proton ou un ion dérivé d'un métal alcalin ou alcalino-terreux,

- n représente un nombre entier égal à 1 ou 2,

- m représente un nombre entier égal à 0, 1 ou 2, et

- p représente un nombre entier égal à 1 ou 2, étant entendu que p + m est inférieur ou égal à 3,

étant entendu que les radicaux R et $R_1$ peuvent être chiraux ou achiraux, et que

- lorsque l'un des radicaux R et $R_1$ est chiral, p et m peuvent être égaux à 1 ou 2

- lorsque les radicaux R et $R_1$ sont achiraux, p et m sont nécessairement égaux à 1, les radicaux R et $R_1$ étant alors différents de façon que la substitution du ou des atome(s) du phosphore du produit de formule générale (I) soit dissymétrique

- lorsque m est égal à 0, les radicaux R sont différents de façon que la substitution de l'atome de phosphore du produit de formule générale (I) soit dissymétrique.

2 - Un procédé de préparation d'une phosphine chirale sulfonée optiquement active selon la revendication 1 caractérisé en ce que l'on fait agir un agent de sulfonation sur une phosphine de formule générale:

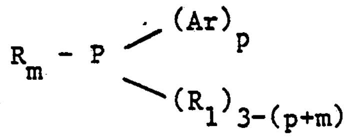

dans laquelle R, $R_1$ Ar sont définis comme dans la revendication 1, n et p étant égaux à 1 ou 2 et p + m étant ⩽ 3, l'un au moins des symboles R et $R_1$ étant chiral.

3 - Procédé selon la revendication 2 caractérisé en ce que l'agent de sulfonation est choisi parmi les oléums, l'acide chlorosulfonique, les dérivés de l'anhydride sulfurique ou le sulfate de bis-triméthylsilyle.

4 - Un procédé de préparation d'une phosphine chirale sulfonée optiquement active selon la revendication caractérisé en ce que l'on fait réagir un agent de sulfonation sur une phosphine de formule générale:

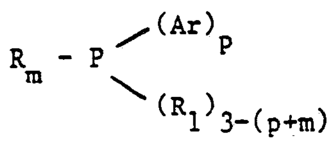

dans laquelle R, $R_1$, Ar sont définis comme précédemment, p et m sont égaux à 1, les symboles R et $R_1$ étant achiraux et le symbole étant différent de $R_1$, puis dédouble la phosphine sulfonée obtenue au moyen d'une base optiquement active.

5 - Un procédé de préparation d'une phosphine chirale sulfonée optiquement active selon la revendication 1 caractérisé en ce que l'on fait réagir un agent de sulfonation sur une phosphine de formule générale:

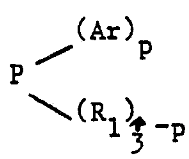

dans laquelle $R_1$, Ar et p sont définis comme dans la revendication 1, les symboles $R_1$ étant différents, puis dédouble la phosphine sulfonée obtenue au moyen d'une base optiquement active.

6 - Utilisation des phosphines chirales sulfonées optiquement actives selon la revendication 1 pour la préparation de cordinats de complexes métalliques catalytiques solubles dans l'eau utilisables pour réaliser sélectivement des synthèses asymétriques en chimie orqanique.

7 - Utilisation des phosphines chirales sulfonées optiquement actives selon la revendication 1 pour la préparation de coordinats de complexes métalliques catalytiques solubles dans l'eau utilisables pour réaliser une hydrogénation asymétrique sélective.

8 - Utilisation des phosphines chirales sulfonées optiquement actives selon la revendication 1 pour la préparation de coordinats de complexes métalliques catalytiques solubles dans l'eau utilisables pour réaliser une hydroformylation asymétrique sélective.

**Revendications** poir l'Etat contractant AT

1 - Procédé de préparation d'une nouvelle phosphine chirale sulfonée optiquement active de formule générale:

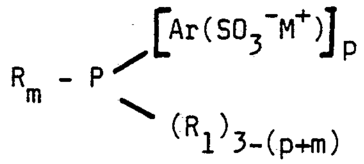

(I)

dans laquelle:

- R représente un radical alcoyle contenant 1 à 12 atomes de carbone en chaîne droite ou ramifiée, cycloalcoyle contenant 3 à 6 atomes de carbone et éventuellement substitué par 1 ou 2 radicaux alcoyles contenant 1 à atomes de carbone, cycloalcoylalcoyle contenant 4 à 10 atomes de carbone et éventuellement substitué par un radical alcoyle contenant 1 à 4 atomes de carbone, phényle ou phénylalcoyle dont la partie alcoyle contient 1 à 4 atomes de carbone, binaphtyle ou binaphtylalcoyle dont la partie alcoyle contient 1 à 4 atomes de carbone, les radicaux alcoyles, cycloalcoyles, phényles, binaphtyles et les portions alcoyles des autres radicaux étant éventuellement substitués par un radical de formule générale:

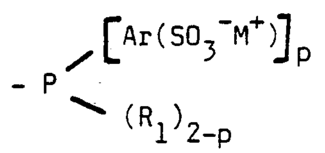

- $R_1$ représente un radical alcoyle contenant 1 à 12 atomes de carbone en chaîne droite ou ramifiée, cycloalcoyle contenant 3 à 6 atomes de carbone, phényle ou phénylalcoyle dont la partie alcoyle contient 1 à 4 atomes de carbone,
- Ar représente un radical phényle ou naphtyle éventuellement substitué par un ou plusieurs radicaux alcoyles ou alcoyloxyles contenant 1 à 4 atomes carbone,
- $M^+$ représente un proton ou un ion dérivé d'un métal alcalin ou alcalinoterreux
- n représente un nombre entier égal à 1 ou 2
- m représente un nombre entier égal à 0, 1 ou 2, et
- p représente un nombre entier égal à 1 ou 2, étant entendu que $p+m$ est inférieur ou égal à 3,

étant entendu que les radicaux $R_1$ et $R_2$ peuvent être chiraux ou achiraux, et que:

- lorsque l'un des radicaux R et $R_1$ est chiral, p et m peuvent être égaux à 1 ou 2
- lorsque les radicaux R et $R_1$ sont achiraux, p et m sont nécessairement égaux à 1, les radicaux R et $R_1$ étant alors différents de façon que la substitution du ou des atome(s) du phosphore du produit de formule générale (1) soit dissymétrique
- lorsque m est égal à 0, les radicaux $R_1$ sont différents de façon que la substitution de l'atome de phosphore du produit de formule générale (I) soit dissymétrique, caractérisé en ce que l'on fait réagir un agent de sulfonation:

soit sur une phosphine de formule générale:

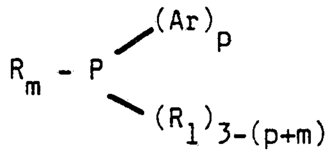

dans laquelle:

ou bien R, $R_1$ et Ar sont définis comme précédemment, n et p étant égaux à 1 ou 2 et p + m étant ⩽ 3, l'un au moins des symboles R et $R_1$ étant chiral,

ou bien R, $R_1$ et Ar sont définis comme précédemment, p + m sont égaux à 1, les symboles R et $R_1$ étant achiraux et le symbole R étant différent de $R_1$, puis dédouble la phosphine sulfonée obtenue au moyen d'une base optiquement active,

soit sur une phosphine de formule générale:

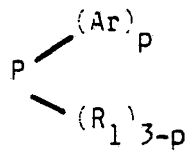

dans laquelle $R_1$, Ar et p sont définis comme précédemment, les symboles $R_1$ étant différents, puis dédouble la phosphine sulfonée obtenue au moyen d'une base optiquement active.

2 - Procédé selon la revendication 1 caractérisé en ce que l'agent de sulfonation est choisi parmi les oléums, l'acide chlorosulfonique, les dérivés de l'anhydride sulfurique ou le sulfate de bis-triméthylsilyle.

3 - Utilisation des phosphines chirales sulfonées optiquement actives obtenues selon le procédé de la revendication 1 pour la préparation des coordinats de complexes catalytiques solubles dans l'eau utilisables pour réaliser sélectivement des synthèses asymétriques en chimie organique.

4 - Utilisation des phosphines chirales sulfonées optiquement actives obtenues selon le procédé de la revendication 1 pour la préparation de coordinats de complexes métalliques catalytiques solubles dans l'eau utilisables pour réaliser une hydrogénation asymétrique sélective.

5 - Utilisation des phosphines chirales sulfonées optiquement actives obtenues selon le procédé de la revendication 1 pour la préparation de coordinats de complexes métalliques catalytiques solubles dans l'eau utilisables pour réaliser une hydroformylation asymétrique sélective.

**Patentansprüche** für die Vertragsstaaten

BE, CH, DE, FR, GB, IT, LU, NL, SE, LI

1. Ein neues optisch aktives chirales sulfoniertes Phosphin, dadurch gekennzeichnet, daß es der allgemeinen Formel

$$R_m - P \begin{cases} [Ar(SO_3^-M^+)_n]_p \\ (R_1)_{3-(p+m)} \end{cases} \qquad (I)$$

entspricht, worin:

- R einen Alkylrest mit 1 bis 12 Kohlenstoffatomen in gerader oder verzweigter Kette bedeutet, Cycloalkyl mit 3 bis 6 Kohlenstoffatomen und gegebenenfalls substituiert durch 1 oder 2 Alkylreste mit 1 bis 4 Kohlenstoffatomen, Cycloalkylalkyl mit 4 bis 10 Kohlenstoffatomen und gegebenenfalls substituiert durch einen Alkylrest mit 1 bis 4 Kohlenstoffatomen, Phenyl oder Phenylalkyl, dessen Alkylteil 1 bis 4 Kohlenstoffatome enthält, Binaphthyl oder Binaphthylalkyl, dessen Alkylteil 1 bis 4 Kohlenstoffatome enthält, wobei die Alkyl-, Cycloalkyl-, Phenyl-, Binaphthylreste und die Alkylteile der anderen Reste, gegebenenfalls durch einen Rest der allgemeinen formel

$$- P \begin{cases} [Ar(SO_3^-M^+)_n]_p \\ (R_1)_{2-p} \end{cases}$$

substituiert sind;

- $R_1$ einen Alkylrest mit 1 bis 12 Kohlenstoffatomen in gerader oder verzweigter Kette, Cycloalkyl mit 3 bis 6 Kohlenstoffatomen, Phenyl oder Phenylalkyl, dessen Alkylteil 1 bis 4 Kohlenstoffatome enthält, bedeutet,
- Ar einen Phenyl- oder Naphthylrest, gegebenenfalls substituiert durch einen oder mehrere Alkyl- oder Alkoxyreste mit 1 bis 4 Kohlenstoffatomen, bedeutet,
- M+ ein Proton oder ein von einem Alkali- oder Erdalkalimetall stammendes Ion bedeutet,
- n eine ganze Zahl gleich 1 oder 2 bedeutet,
- m eine ganze Zahl gleich 0, 1 oder 2 bedeutet, und
- p eine ganze Zahl gleich 1 oder 2 bedeutet, wobei p + m unterhalb oder gleich 3 ist,

wobei die Reste R und $R_1$ chiral oder achiral sein können, und daß

- wenn einer der Reste R und $R_1$ chiral ist, p und m gleich 1 oder 2 sein können,
- falls die Reste R und $R_1$ achiral sind, p und m notwendigerweise gleich 1 sind, wobei die Reste R und $R_1$ dann verschieden sind, derart, daß die Substitution des Atoms oder der Atome des Phosphors des Produkts der allgemeinen Formel Iunsymmetrisch ist,
- falls m gleich 0 ist, die Reste R verschieden sind, derart, da- die Substitution des Phosphoratoms des Produkts der allgemeinen Formel I unsymmetrisch ist.

2. Verfahren zur Herstellung eines chiralen sulfonierten optisch aktiven Phosphins gemäß Anspruch 1, dadurch gekennzeichnet, daß man ein Sulfonierungsmittel auf ein Phosphin der allgemeinen Formel

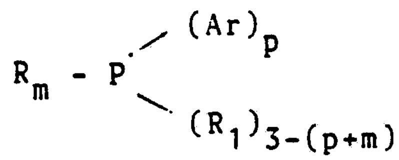

einwirken läßt, worin R, $R_1$, Ar wie in Anpsruch 1 definiert sind, n und p gleich 1 oder 2 sind, und $p + m \leqslant 3$ ist, und wenigstens eines der Symbole R und $R_1$ chiral ist.

3. Verfahren gemäß Anspruch 2, dadurch gekennzeichnet, daß das Sulfonierungsmittel ausgewählt ist unter Oleum, Chlorsulfonsäure, den Derivaten des Schwefelsäureanhydrids oder Bis-trimethylsilylsulfat.

4. Verfahren zur Herstellung eines chiralen sulfonierten optisch aktiven Phosphins gemäß Anspruch 1, dadurch gekennzeichnet, daß man ein Sulfonierungsmittel auf ein Phosphin der allgemeinen Formel

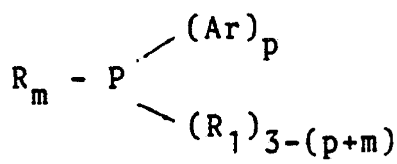

einwirken läßt, worin R, $R_1$, Ar wie vorstehend definiert sind, p und m gleich 1 sind, die Symbole R und $R_1$ achiral sind und das Symbol R verschieden von $R_1$ ist, daß man dann das erhaltene sulfonierte Phosphin mittels einer optisch aktiven Base auftrennt.

5. Verfahren zur Herstellung eines chiralen sulfonierten optisch aktiven Phosphins gemäß Anspruch 1, dadurch gekennzeichnet, daß man ein Sulfonierungsmittel auf ein Phosphin der allgemeinen Formel

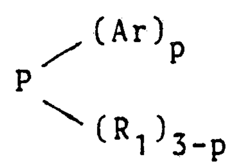

einwirken läßt, worin $R_1$ Ar und p wie in Anspruch 1 definiert sind und die Symbole $R_1$ verschieden sind daß man dann das erhaltene sulfonierte Phosphin mittels einer optisch aktiven Base auftrennt.

6. Verwendung der chiralen sulfonierten optisch aktiven Phosphine gemäß Anspruch 1 zur Herstellung von Koordinaten von wasserlöslichen katalytischen Metallkomplexen, die zur selektiven Durchführung von asymmetrischen Synthesen in der organischen Chemie verwendbar sind.

7. Verwendung der chiralen sulfonierten optisch aktiven Phosphine gemäß Anspruch 1 zur Herstellung von Koordinaten von wasserlöslichen katalytischen Metallkomplexen zur Durchführung einer selektiven asymmetrischen Hydrierung.

8. Verwendung der chiralen sulfonierten optisch aktiven Phosphine gemäß Anspruch 1 zur Herstellung von Koordinaten von wasserlöslichen katalytischen Metallkomplexen zur Durchführung einer selektiven asymmetrischen Hydroformylierung.

**Patentansprüche** für den Vertragsstaat AT

1. Verfahren zur Herstellung eines neuen optisch aktiven sulfonierten chiralen Phosphins der allgemeinen Formel:

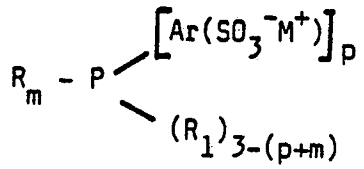

(I)

in welcher:

- R bedeutet einen gerad- oder verzweigtkettigen Alkylrest mit 1 bis 12 Kohlenstoffatomen, einen Cycloalkylrest mit 3 bis 6 Kohlenstoffatomen, gegebenenfalls substituiert durch 1 oder 2 Alkylreste mit 1 bis 4 Kohlenstoffatomen, einen Cycloalkylalkylrest mit 4 bis 10 Kohlenstoffatomen, gegebenenfalls substituiert durch einen Alkylrest mit 1 bis 4 Kohlenstoffatomen, einen Phenylrest oder einen Phenylalkylrest, dessen Alkylteil 1 bis 4 Kohlenstoffatome enthält, einen Binaphthylrest oder einen Binaphthylalkylrest, dessen Alkylteil 1 bis 4 Kohlenstoffatome enthält, wobei die Alkyl-, Cycloalkyl-, Phenyl-, Binaphthylreste und die Alkylteile der übrigen Reste gegebenenfalls durch einen Rest der allgemeinen Formel:

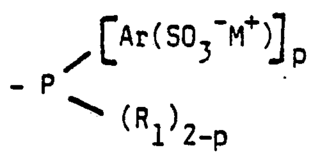

substituiert sind

- $R_1$ bedeutet einen gerad- oder verzweigtkettigen Alkylrest mit 1 bis 12 Kohlenstoffatomen, einen Cycloalkylrest mit 3 bis 6 Kohlenstoffatomen, einen Phenylrest oder einen Phenylalkylrest, dessen Alkylteil 1 bis 4 Kohlenstoffatome enthält,
- Ar bedeutet einen gegebenenfalls durch ein oder mehrere Alkyl- oder Alkoxyreste mit 1 bis 4 Kohlenstoffatomen substituierten Phenyl- oder Naphthylrest,
- $M+$ bedeutet ein Proton oder ein von einem Alkali- oder Erdalkalimetall abgeleitetes Ion
- n bedeutet eine ganze Zahl gleich 1 oder 2
- m bedeutet eine ganze Zahl gleich 0, 1 oder 2 und
- p bedeutet eine ganze Zahl gleich 1 oder 2, wobei selbstverständlich $p + m$ kleiner oder gleich 3 ist, und die Reste $R_1$ und $R_2$ selbstverständlich chiral oder achiral sein können und:
- wenn einer der Reste R und $R_1$ chiral ist, p und m gleich 1 oder 2 sein können
- wenn die Reste R und $R_1$ achiral sind, p und m notwendigerweise gleich 1 sind, wobei die Reste R und $R_1$ dann derart verschieden sind, daß die Substitution des Phosphoratoms oder der Phosphoratome des Produktes der allgemeinen Formel (I) asymmetrisch ist,
- wenn m gleich 0 ist, sind die Reste $R_1$ derart verschieden, daß die Substitution des Phosphoratoms des Produktes der allgemeinen Formel (I) asymmetrisch ist, dadurch gekennzeichnet, daß man ein Sulfonierungsmittel:

entweder mit einem Phosphin der allgemeinen Formel:

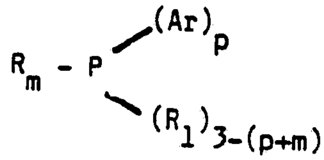,

in welcher
entweder R, $R_1$ und Ar wie oben definiert sind, n und p gleich 1 oder 2 sind und $p + m \leqslant 3$ ist, wobei mindestens eines der Symbole R und $R_1$ chiral ist,
oder R, $R_1$ und Ar wie oben definiert sind, p + m gleich 1 ist, die Symbole R und $R_1$ achiral sind und das Symbol R von $R_1$ verschieden ist, umsetzt, wobei das erhaltene sulfonierte Phosphin mittels einer optisch aktiven Base gespalten wird, oder mit einem Phosphin der allgemeinen Formel:

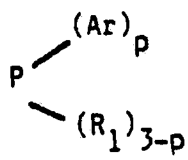,

in welcher $R_1$, Ar und p wie oben definiert sind, wobei die Symbole $R_1$ verschieden sind, umsetzt, worauf das erhaltene sulfonierte Phosphin mittels einer optisch aktiven Base gespalten wird.

2. Verfahren nach Anspruch 1, dadurch gekennzeichnet, daß das Sulfonierungsmittel ausgewählt wird aus rauchender Schwefelsäure, Chlorosulfonsäure, den Derivaten von Schwefelsäureanhydrid oder Bis-trimethylsilylsulfat.

3. Verwendung von nach dem Verfahren nach Anspruch 1 erhaltenen optisch aktiven sulfonierten chiralen Phosphinen zur Herstellung von Liganden wasserlöslicher katalytischer Komplexe, die zur selektiven Durchführung von asymmetrischen Synthesen in der organischen Chemie einsetzbar sind.

4. Verwendung von nach dem Verfahren nach Anspruch 1 erhaltenen optisch aktiven sulfonierten chiralen Phosphinen zur Herstellung von Liganden wasserlöslicher katalytischer Metallkomplexe, die zur Durchführung einer selektiven asymmetrischen Hydrierung einsetzbar sind.

5. Verwendung von nach dem Verfahren nach Anspruch 1 erhaltenen optisch aktiven sulfonierten chiralen Phosphinen zur Herstellung von Liganden wasserlöslicher katalytischer Metallkomplexe, die zur Durchführung einer selektiven asymmetrischen Hydroformylierung einsetzbar sind.

**Claims** for the Contracting States BE, CH, DE, FR, GB, IT, LU, NL, SE, LI

1. A new, optically active, sulphonated, chiral phosphine, characterized in that it corresponds to the general formula:

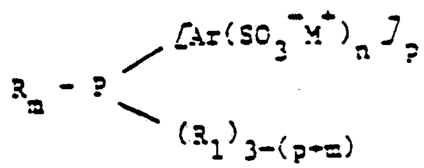

(I)

in which:

- R denotes an alkyl radical containing 1 to 12 carbon atoms as a straight or branched chain, a cycloalkyl radical containing 3 to 6 carbon atoms and, if desired, substituted by 1 or 2 alkyl radicals containing 1 to 4 carbon atoms, a cycloalkylalkyl radical containing 4 to 10 carbon atoms and, if desired, substituted by an alkyl radical containing 1 to 4 carbon atoms, a phenyl or phenylalkyl radical in which the alkyl moiety contains 1 to 4 carbon atoms, a binaphthyl or binaphthylalkyl radical in which the alkyl moiety contains 1 to 4 carbon atoms, the alkyl, cycloalkyl, phenyl and binaphthyl radicals and the alkyl moieties of the other radicals being substituted, if desired, by a radical of general formula:

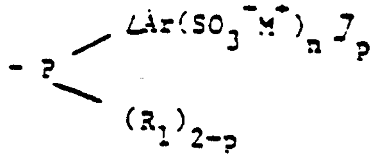

- $R_1$ denotes an alkyl radical containing 1 to 12 carbon atoms as a straight or branched chain, a cycloalkyl radical containing 3 to 6 carbon atoms, or a phenyl or phenylalkyl radical in which the alkyl moiety contains 1 to 4 carbon atoms,
- Ar denotes a phenyl or naphthyl radical substituted, if desired, by one or more alkyl or alkoxy radicals containing 1 to 4 carbon atoms,
- M+ denotes a proton or an ion derived from an alkali metal or alkaline-earth metal,
- n denotes an integer equal to 1 or 2,
- m denotes an integer equal to 0, 1 or 2, and
- p denotes an integer equal to 1 or 2, it being understood that p + m is less than or equal to 3, it being understood that the radicals R and $R_1$ may be chiral or achiral, and that
- when one of the radicals R and $R_1$ is chiral, p and m may be equal to 1 or 2
- when the radicals R and $R_1$ are achiral, p and m are necessarily equal to 1, the radicals R and $R_1$ then being different so that the substitution of the phosphorus atom(s) of the product of general formula (I) be dissymmetrical
- when m is equal to 0, the radicals $R_1$ are different so that the substitution of the phosphorus atom of the product of general formula (I) be dissymmetrical.

2. A process for the preparation of an optically active, sulphonated, chiral phosphine according to Claim 1, characterized in that a sulphonating agent is caused to react with a phosphine of general formula:

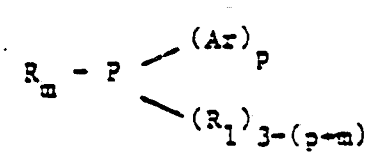

in which R, $R_1$ and ar are defined as in Claim 1, n and p being equal to 1 or 2, and p + m being ⩽ 3, at least one of the symbols R and R1 being chiral.

3. Process according to Claim 2, characterized in that the sulphonating agent is chosen from oleums, chlorosulphonic acid, sulphur trioxide derivatives or bistrimethylsilyl sulphate.

4. A process for the preparation of an optically active, sulphonated, chiral phosphine according to Claim 1, characterized in that a sulphonating agent is caused to react with a phosphine of general formula:

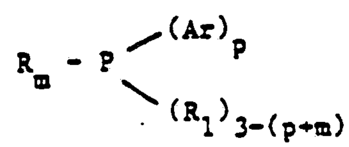

in which R, $R_1$ and Ar are defined as above, p and m are equal to 1, the symbols R and $R_1$ being achiral and the symbol R being other than $R_1$, and the sulphonated phosphine obtained is then resolved by means of an optically active base.

5. A process for the preparation of an optically active, sulphonated, chiral phosphine according to Claim 1, characterized in that a sulphonating agent is caused to react with a phosphine of general formula:

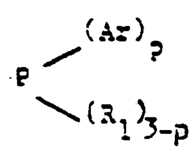

in which $R_1$, Ar and p are defined as in claim 1, the symbols $R_1$ being different, and the sulphonated phosphine obtained is then resolved by means of an optically active base.

6. Use of the optically active, sulphonated, chiral phosphine according to Claim 1, for the preparation of coordinates of water-soluble catalytic metal complexes which can be used to accomplish asymmetric syntheses selectively in organic chemistry.

7. Use of the optically active, sulphonated, chiral phosphines according to Claim 1 for the preparation of coordinates of water-soluble catalytic metal complexes which can be used to accomplish a selective asymmetric hydrogenation.

8. Use of the optically active, sulphonated, chiral phosphines according to Claim 1 for the preparation of coordinates of water-soluble catalytic metal complexes which can be used to accomplish a selective asymmetric hydroformylation.

**Claims** for the Contracting State AT

1. Process for the preparation of a new optically active, sulphonated, chiral phosphine of general formula:

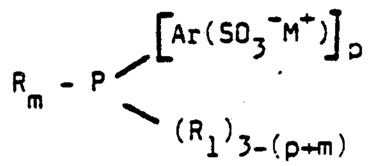

(I)

in which:

- R denotes an alkyl radical containing 1 to 12 carbon atoms as a straight or branched chain, a cycloalkyl radical containing 3 to 6 carbon atoms and, if desired, substituted by 1 or 2 alkyl radicals containing 1 to 4 carbon atoms, a cycloalkylalkyl radical containing 4 to 10 carbon atoms and, if desired, substituted by an alkyl radical containing 1 to 4 carbon atoms, a phenyl or phenylalkyl radical in which the alkyl moiety contains 1 to 4 carbon atoms, a binaphthyl or binaphthylalkyl radical in which the alkyl moiety contains 1 to 4 carbon atoms, the alkyl, cycloalkyl, phenyl and binaphthyl radicals and the alkyl moieties of the other radicals being substituted, if desired, by a radical of general formula:

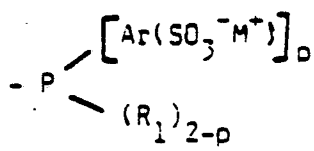

- $R_1$ denotes an alkyl radical containing 1 to 12 carbon atoms as a straight or branched chain, a cycloalkyl radical containing 3 to 6 carbon atoms, or a phenyl or phenylalkyl radical in which the alkyl moiety contains 1 to 4 carbon atoms,
- Ar denotes a phenyl or naphthyl radical substituted if desired by one or more alkyl or alkoxy radicals containing 1 to 4 carbon atoms,
- $M^+$ denotes a proton or an ion derived from an alkali metal or alkaline-earth metal,
- n denotes an integer equal to 1 or 2,
- m denotes an integer equal to 0, 1 or 2, and
- p denotes an integer equal to 1 or 2, it being understood that p + m is less than or equal to 3, it being understood that the radicals $R_1$ and $R_2$ may be chiral or achiral, and that
- when one of the radicals R and $R_1$ is chiral, p and m may be equal to 1 or 2
- when the radicals R and $R_1$ are achiral, p and m are necessarily equal to 1, the radicals R and $R_1$ then being different so that the substitution of the phosphorus atom(s) of the product of general formula (I) be dissymmetrical
- when m is equal to 0, the radicals $R_1$ are different so that the substitution of the phosphorus atom of the product of general formula (I) be dissymmetrical, characterized in that a sulphonating agent is caused to react:

either with a phosphine of general formula:

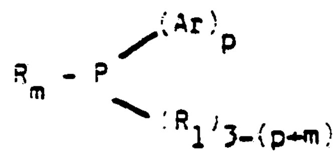

in which:

either R, $R_1$ and Ar are defined as above, n and p being equal to 1 or 2 and p + m being $\leqslant$ 3, at least one of the symbols R and $R_1$ being chiral,

or R, $R_1$ and ar are defined as above, p + m are equal. to 1, the symbols R and $R_1$ being achiral and the symbol R being other than $R_1$, and the sulphonated phosphine obtained is then resolved by means of an optically active base,

or with a phosphine of general formula:

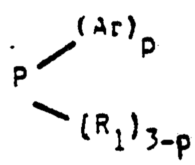

in which $R_1$, Ar and p are defined as above, the symbols $R_1$ being different, and the sulphonated phosphine obtained is then resolved by means of an optically active base.

2. Process according to claim 1, characterized in that the sulphonating agent is chosen from oleums, chlorosulphonic acid, sulphur trioxide derivatives or bistrimethylsilyl sulphate.

3. Use of the optically active, sulphonated, chiral phosphines obtained according to the process of claim 1, for the preparation of coordinates of water-soluble catalytic complexes which can be used to accomplish asymmetric syntheses selectively in organic chemistry.

4. Use of the optically active, sulphonated, chiral phosphines obtained according to the process of claim 1, for the preparation of coordinates of water-soluble catalytic metal complexes which can be used to accomplish a selective asymmetric hydrogenation.

5. Use of the optically active, sulphonated, chiral phosphines obtained according to the process of claim 1, for the preparation of coordinates of water-soluble catalytic metal complexes which can be used to accomplish a selective asymmetric hydroformylation.